# EUROPEAN PATENT SPECIFICATION

(11) **EP 0 500 177 B1**
(45) Date of publication and mention of the grant of the patent: **07.01.1999**
(21) Application number: 92200431.2
(22) Date of filing: 15.02.1992
(51) Int. Cl.: C07D 263/14, C07D 263/10, C07C 323/32

(54) **Process for the preparation of trans-(5R)-2,4,5-trisubstituted 2-oxazolines**
Verfahren zur Herstellung von trans-(5R)-2,4,5-trisubstituierten 2-Oxazolinen
Procédé de préparation de trans-(5R-)oxazolines 2,4,5-trisubstitués

(30) Priority: 21.02.1991 IT MI910457
(43) Date of publication of application: 26.08.1992
(73) Proprietor: ZAMBON GROUP S.p.A., I-36100 Vicenza (IT)
(72) Inventor: Villa, Marco, I-20131 Milano (IT); Giordano, Claudio, I-20052 Monza (Milano) (IT); Paiocchi, Maurizio, I-20152 Milano (IT)
(74) Representative: Marchi, Massimo, Dr.

(56) References cited:
- EP-A- 130 633
- DE-C- 940 897
- FR-A- 1 071 077

## Description

This invention relates to a process for the preparation of 2,4,5-trisubstituted 2-oxazoline compounds having trans-(5R) configuration.

More particularly, this invention relates to a process for the preparation of the above compounds from precursors wherein the carbon atom that will be at position 5 in the oxazoline ring, has S configuration.

(2R,3R)-3-(4-methylsulfonylphenyl)-2-dichloroacetamido-1,3-propanediol is an antibiotic known as Thiamphenicol (Merck Index XI Ed., n. 9230, p. 1465).

(2S,3R)-3-(4-methylsulfonyl)-3-hydroxy-2-dichloroacetamido-1-fluoro-propane is an antibiotic known as Florfenicol (Merck Index XI Ed., n. 4042, p. 642).

(2R,3R)-3-(4-methylthiophenyl)-2-amino-1,3-propanediol (hereinafter referred to as 2R,3R-thiomicamine) and (2S,3R)-3(4-methylthiophenyl)-3-hydroxy-2-amino-1-fluoropropane (hereinafter referred to as 2S,3R-fluorothiomicamine) as well as their N-dichloroacetyl derivatives are intermediate compounds in the synthesis of Thiamphenicol and Florfenicol, respectively.

It is worthy of notice that the absolute configuration of the above mentioned two compounds is analogous while the apparent difference in the configuration of the atom at position 2 is due exclusively to rules of nomenclature.

The literature about the synthesis of Thiamphenicol and Florfenicol is rich in publications and patents.

The British patent 745 900 discloses the preparation of Thiamphenicol from 2R,3R-thiomicamine via N-dichloroacetylation and oxidation of the CH₃S group to a CH₃SO₂ group.

EP-B-014 437 (Schering Corp.) discloses the preparation of Florfenicol from 2S,3R-fluorothiomicamine by oxidation of the group CH₃S to a CH₃SO₂ group and subsequent N-dichloroacetylation. This patent also describes a process for the preparation of 2S-3R-fluorothiomicamine.

EP-A-0 130 633 (Zambon S.p.A.) discloses a procedure for the preparation of (2S,3R)fluorothiomicamine by fluorination of heterocyclic compounds prepared by concomitant protection of both the benzyl hydroxy and amino groups of 2R,3R-thiomicamine. Typical examples of said heterocyclic compounds are the (4R,5R)-2-oxazolines substituted at 4 position by a CH₂OH group which is converted into an alkyl- or aryl-sulfonyloxymethyl group (by reaction with a derivative of an alkyl- or aryl-sulfonic acid) and then into a fluoromethyl group, for instance by reaction with KF in a poly glycol. 2-oxazolines must have 4R,5R configuration, i.e. the proper configuration necessary to give the desired 2S,3R-fluorothiomicamine, after hydrolyzation.

The Italian patent 1 216 880 (Zambon Group S.p.A.) discloses the preparation of the 2-phenyl-4-sulfonyloxymethyl substituted 4R,5R-oxazolines of EP-A-0 130 633, by reacting (2R,3R)-N-benzoyl-thiomicamine with the chloride of a sulfonic acid in the presence of a base.

PCT WO 86/017799 (Schering Corp.) discloses a process for preparing the racemic threo-fluorothiomicamine and the optical resolution thereof to give the (2S,3R) enantiomer.

The Italian patent 1 223 563 (Zambon Group S.p.A.) discloses a process for converting 2S,3S-thiomicamine into an equimolar racemic mixture of 2S,3S and 2R,3R-thiomicamine.

The Italian patent 1 216 538 (Zambon Group S.p.A.) discloses a process for preparing the 2-phenyl-4-sulfonyloxymethyl substituted 4R,5R-oxazolines of EP-A-0 130 633, by reacting (2R,3R)-thiomicamine with benzonitrile.

U.S. patent 4 876 352 (Schering Corp.) discloses a process for fluorinating 2-oxazolines or the forms thereof wherein the sulfur has been oxidated, of EP-A-0 130 633.

PCT WO 90/14434 (Schering Corp.) discloses a process for the enzymatic separation of precursors of Thiamphenicol and Florfenicol and their conversion into Thiamphenicol and Florfenicol through known reactions and intermediates such as, for example, the 2-oxazolines described in EP-A-0 130 633.

EP-A-0 423 705 (Zambon Group S.p.A.), discloses a process for the inversion of 2S,3S-thiomicamine into the desired 2R,3R enantiomer. Some intermediates of this process can be converted into 2S,3R-fluorothiomicamine which is useful for preparing Florfenicol.

The above review clearly shows that (4R,5R)-2-oxazolines of EP-A-0 130 633 are key intermediates in the preparation of Florfenicol because they may be easily fluorinated and give, after hydrolyzation, threo-(2S,3R)-fluorothiomicamine. Indeed, the hydrolyzation of non-fluorinated oxazolines affords threo-(2R,3R)-thiomicamine.

The literature also reports on the use of the threo-(2S,3S) enantiomer of thiomicamine in the synthesis of Thiamphenicol or Florfenicol, via racemization or complete inversion of its configuration.

The literature does not teach, however, how to use the erythro enantiomers of thiomicamine or derivatives thereof in the synthesis of Thiamphenicol or Florfenicol.

Surprisingly, it has been now found a process for the preparation of a 2-oxazoline compound of the formula 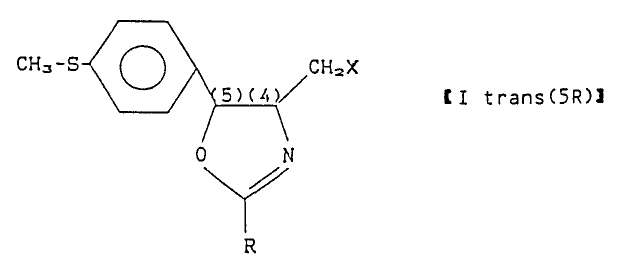 wherein
- R: is alkyl, alkoxy, alkyl substituted by 1 or 2 substituents selected from the group comprising halogens and C₁-C₄ alkoxy, alkenyl, alkenyl substituted by 1 or 2 substituents selected from the group comprising halogens and C₁-C₄ alkoxy, phenyl, phenyl substituted by 1 or 2 substituents selected from the group comprising halogens, C₁-C₄ alkoxy, C₁-C₄ alkyl, cyano, nitro and trifluoromethyl, phenylalkyl or phenylalkyl substituted by 1 or 2 substituents selected from the group comprising halogens, C₁-C₄ alkoxy, C₁-C₄ alkyl, cyano, nitro and trifluoromethyl, and
- X: is hydroxy, halogen or acyloxy from a carboxylic or sulfonic acid,
comprising treating a compound of the formula 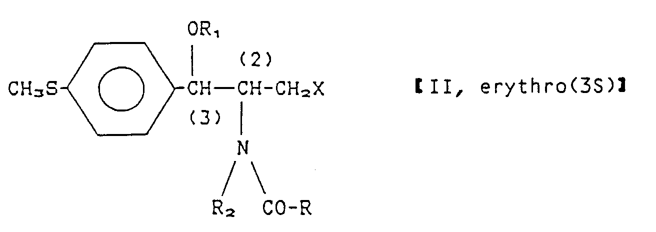 wherein
- R and X: have the above mentioned meanings,
- R₁: is hydrogen or acyl,
- R₂: is hydrogen, or
- R₁ and R₂: together form a group 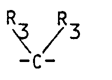 where each R₃ is hydrogen, alkyl, alkoxy or phenyl, or both R₃ together form a tetra or pentamethylene chain.
with an ionizing non-nucleophilic agent selected from strong protic acids and Lewis acids having affinity for oxygen, in a substantially anhydrous inert solvent or diluting agent, at a temperature of from -20 to 100°C.

Compounds of formula I are known from EP-A-0 130 633.

Compounds of formula II are erythro stereoisomers of known compounds deriving from threo-thiomicamine.

In the above formulas
- halogen means fluorine, chlorine, bromine or iodine;
- alkyl means a linear or branched alkyl preferably having from 1 to 6 carbon atoms; preferred meanings are methyl, ethyl, n-propyl, isopropyl and n-butyl;
- alkenyl means a linear or branched chain having 2-6 carbon atoms and at least one double bond; preferred meanings are vinyl, allyl, 1-propenyl, 1-butenyl, isobutenyl and 1-hexenyl;
- phenylalkyl preferably means benzyl and phenyl;
- substituted alkyl and substituted alkenyl, mean the above mentioned alkyl and alkenyl radical substituted by 1 or 2 substituents preferably selected from the group comprising halogens (in particular F, Cl and Br) and C₁-C₄ alkoxy;
- substituted phenyl and substituted phenylalkyl mean phenyl and the above mentioned phenylalkyl radical substituted by 1 or 2 substituents preferably selected from the group comprising halogens, C₁-C₄ alkoxy, C₁-C₄ alkyl, cyano, nitro and trifluoromethyl;
- acyl of a carboxylic acid means the acyl residue of an optionally substituted carbonic or carboxylic C₁-C₄ aliphatic acid; preferred meanings are acetyl, propionyl, dichloroacetyl, phenylacetyl, methoxyacetyl, and benzoyl optionally substituted by 1 or 2 substituents preferably selected from the group comprising halogens, C₁-C₄ alkyl and C₁-C₄ alkoxy;
- acyl of a sulfonic acid means the acyl residue (i) of an alkylsulfonic acid; preferred meanings are methanesulfonyl, ethanesulfonyl and trifluoromethanesulfonyl, or (ii) of an arylsulfonic acid; preferred meanings are benzenesulfonyl, p-toluenesulfonyl and naphthalenesulfonyl.

A preferred class of compounds of formula II is that wherein both R₁ and R₂ are hydrogen. A most preferred group of compounds of formula II is that wherein both R₁ and R₂ are hydrogen and R is alkyl, substituted alkyl, phenyl, and substituted phenyl. Preferred meanings of R are methyl, ethyl, dichloro-methyl, phenyl, 4-tolyl, 2,6-dimethyl-phenyl, 2,6-dichloro-phenyl, 4-nitro-phenyl, 4-chloro-phenyl and 4-methoxy-phenyl.

Another preferred class of compounds of formula II is that wherein R₁ and R₂ together form a group 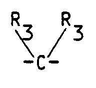 where both R₃ are methyl, ethyl, phenyl, or together form a tetramethylene or a pentamethylene chain.

In both the preferred classes of the compounds of formula II, the preferred meanings of X are fluoro, hydroxy, acetoxy, benzoyloxy, propanoyloxy, methanesulfonyloxy, ethanesulfonyloxy, benzenesulfonyloxy and p-toluenesulfonyloxy.

The process of this invention is carried out by bringing into contact a compound of formula II with an ionizing non-nucleophilic agent.

The expression "ionizing agent" is herein intended to mean an agent capable of increasing the polarity of a covalent bond between two different atoms (Jerry March, Advanced Organic Chemistry, III Ed., 1985, p.318).

In the specific case of this invention, the two different atoms are the benzylic carbon at position 3 and the oxygen atom linked thereto.

In turn, the expression "non-nucleophilic agent" is herein intended to mean an agent which does not have the reactivity of nucleophiles, i.e. an agent which does not tend to give substitution reactions to the benzylic carbon at position 3.

Specific examples of ionizing non-nucleophilic agents according to this invention comprise strong protic acids and Lewis acids having affinity for oxygen.

Preferred examples are trifluoroacetic acid, sulfuric acid, methanesulfonic acid, p-toluenesulfonic acid, trifluoromethanesulfonic acid, AlCl₃, and protonated tertiary amines.

When R₁ = H, a preferred embodiment comprises treating compound II with methanesulfonyl chloride and a tertiary amine.

It is likely that a labile intermediate is formed wherein the hydroxy group at position 3 is converted into a mesyloxy group and that the thus obtained protonated tertiary amine acts as the ionizing non-nucleophilic agent.

Solvents or diluents suitable for performing the process of this invention are all the solvents which do not decrease in any significant way the ionizing strength of the system. In particular, suitable solvents and diluents are the non basic ones. Specific examples are the chlorinated hydrocarbons such as dichloromethane, chloroform, 1,1-dichloroethane, 1,2-dichloroethane and chlorobenzene, aromatic hydrocarbons such as benzene, toluene and xylene, and ethers such as diethyl ether, dimethoxyethane and tetrahydrofuran.

The process of this invention is carried out in a substantially anhydrous medium obtained, for instance, by adding a small amount of acetic anhydride to the solvent.

The reaction is preferably carried out at a temperature of from 0 to 40°C.

All the compounds of formula II can be prepared from the erythro 2R,3S thiomicamine according to conventional procedures known in connection with the preparation of the threo stereoisomers of compounds of formula II from threo thiomicamine.

As far as the operative details are concerned reference is made to the above cited references as well as to the specific examples which follow.

Starting from the compounds of formula II wherein X is acyloxy or halogen, the corresponding compounds of formula I are obtained wherein X is acyloxy or halogen, respectively.

Although it is possible to perform the process of this invention from compounds of formula II wherein X is hydroxy, it is however preferable to convert them into the corresponding analogues wherein X is an acyloxy radical from a carboxylic acid. As an alternative, and that can be particularly useful when preparing Florfenicol, one mole of compound II wherein X = OH is reacted with two moles of a sulfonyl halide, thus yielding directly the compounds I wherein X is an acyloxy radical from a sulfonic acid.

The possible presence of an oxazoline stereoisomer having trans-(5S) configuration is not an obstacle since the latter affords the desired trans-(5R) isomer when treated according to the process of this invention.

The significance of this invention lies in the fact that it makes it possible using an erythro isomer of thiomicamine or a derivative thereof having 2R,3S configuration in the preparation of Thiamphenicol and Florfenicol.

This was not possible before this invention.

Indeed, in an analogous manner it is also possible to utilize a mixture of thiomicamine (or derivatives thereof) having erythro 2R,3S and threo 2R,3R configuration, as starting product. It is in fact known that the latter is also converted into an oxazoline I having 4R,5R configuration.

Oxazoline I can be easily converted into Thiamphenicol or Florfenicol by known methods already described in the literature.

By way of example, if one wishes to prepare Thiamphenicol from a compound of formula I, a compound I wherein X is hydroxy or acyloxy is converted, by hydrolyzation, into (2R,3R)-thiomicamine which, as described in the literature, is converted into Thiamphenicol by means of the N-dichloroacetylation and oxidation of the group SCH₃ to SO₂CH₃. As an alternative, a compound of formula I is oxidized at the sulfur atom to yield the corresponding oxazoline compound wherein the phenyl ring at position 5 is substituted by a CH₃SO₂ group, and then undergoes hydrolyzation and N-dichloroacetylation.

On the other hand, if one wishes to prepare Florfenicol the key product is the oxazoline I wherein X is fluorine.

This oxazoline can be prepared from the corresponding compound II or by fluorination of a compound I wherein X is an acyloxy radical from a sulfonic acid, as taught by EP-A-0 130 633, or still it can be prepared by fluorination of an oxazoline I wherein X = OH, according to the method taught by EP-B-0 014 437.

Hydrolyzation of a compound of formula I wherein X is fluorine yields (2S,3R)-3-(4-methylthiophenyl)-3-hydroxy2-amino-1-fluoro-propane.

The conversion of this compound into Florfenicol is taught by U.S. patent 4 235 892.

As an alternative, the oxidation at the sulfur atom of the oxazoline I yields the corresponding oxazoline wherein the phenyl ring at position 5 is substituted by a CH₃SO₂ group. The fluorination and hydrolyzation of the latter oxazoline compound yields (2S,3R)-3-(4-methylsulfonylphenyl)-3-hydroxy-2-amino-1-fluoro-propane which affords, via N-dichloroacetylation, Florfenicol.

Depending on the meaning of X, when R is dichloromethyl in the compound of formula I, partial hydrolyzation affords (2R,3R)-3-(4-methylthiophenyl)-2-dichloroacetamido-1,3-propanediol or (2S,3R)-3-(4-methylthiophenyl)-3-hydroxy-2-dichloroacetamide-1-fluoro-propane.

In the latter compounds, oxidation of the methylthio group to methylsulfonyl yields Thiamphenicol and Florfenicol, respectively.

The following examples are given with the purpose of better illustrating this invention.

### Example 1

### (4R,5R)-2-methyl-4-methanesulfonytoxymexhyl-5-(4-methylthiophenyl)-2-oxazoline (Compound A)

Mesyl chloride (1.83 g; 16.07 mmoles) was added dropwise, under stirring, to a suspension of erythro (2R,3S)-N-acetyl-2-amino-3-[(4-methylthio)-phenyl]-1,3-propanediol (2 g; 7.84 mmoles) in methylene chloride (40 ml) and triethylamine (2 g; 20 mmoles) at 0°C. After 30 minutes the reaction mixture was poured into a saturated solution of sodium bicarbonate and poured into water (40 ml). The organic phase was extracted with methylene chloride, dried over sodium sulfate and evaporated to dryness under vacuum.

2.5 g of Compound A were obtained.
¹H-NMR (300 MHz, CDCl₃) delta (ppm): 2.09 (d, J=1.2 Hz, 3H); 2.47 (s, 3H); 3.03 (s, 3H); 4.15-4.22 (m, 1H); 4.28-4.40 (m, 2H); 5.26 (d, J=6.4 Hz, 1H); 7.17-7.26 (4H, aromatics).

### Example 2

### (4S,5R)-2-methyl-4-fluoromethyl-5-(4-methylthio-phenyl)-2-oxazoline (Compound B)

A mixture of KF (3.0 g; 52.2 mmoles) and PEG 400 (21.2 g) was heated under stirring to 130°C and kept under vacuum for 3.5 hours. After cooling to 110°C, Compound A (7.0 g; 22.2 mmoles) was added to the reaction mixture. The suspension was kept under vacuum at 110°C for 1.5 hours, the vacuum was released and the mixture was kept under stirring at 110°C for additional 25 hours.

The solution was cooled to room temperature, then water (100 ml) and methylene chloride (200 ml) were added. The two phases were separated and the organic phase was washed four times with water (50 ml x 4). The organic phase was dried over sodium sulfate and evaporated to dryness under vacuum. A residue was obtained consisting mainly of Compound B.
¹H-NMR (300 MHz, CDCl₃) delta (ppm): 2.1 (d, J=1.2 Hz, 3H); 2.47 (s, 3H); 4.13 (ddddq, J=5.2 Hz, J=3.9 Hz, J=1.2 Hz, J=7.2 Hz, J=23 Hz, 1H); 4.52 (ddd, J=5.2 Hz, J=10.2 Hz, J=47 Hz, 1H); 4.58 (ddd, J=3.9 Hz, J=10.2 Hz, J=47 Hz, 1H); 5.32 (d, J=7.2 Hz, 1H); 7.18-7.28 (4H, aromatics).

### Example 3

### (2S,3R)-3-(4-methylthiophenyl)-3-hydroxy-2-amino-1-fluoropropane (Compound C)

A mixture of Compound B (prepared as described in Example 2 (2.39 g; 10 mmoles) in water (3 ml) and 36% hydrochloric acid (5.7 g) was heated at 95°C for 3 hours. The mixture was cooled to 20°C, made alkaline to pH 8 with sodium bicarbonate and extracted with methylene chloride (50 ml x 2). The combined organic phases, after drying and evaporation to dryness under vacuum, yielded 2 g of a crude residue containing 70% of Compound C.

A sample gave pure Compound C by crystallization from ethyl acetate.
¹H-NMR (300 MHz, DMSO+D₂O) delta (ppm): 2.46 (s, 3H); 2.93 (dddd, J=16.8 Hz, J=5.86 Hz, J=5.6 Hz, J=4.92 Hz, 1H); 4.15 (J=48 Hz, J=8.8 Hz, J=5.86 Hz, 1H); 4.3 (J=48 Hz, J=8.8 Hz, J=5.6 Hz, 1H); 4.44 (d, J=4.9 Hz, 1H); 7.20-7.30 (4H, aromatics).

### Example 4

### Florfenicol

Sodium tungstate dihydrate (6.4 mg) and 39% hydrogen peroxide (2.8 ml, about 28 mmoles) were added to a solution of crude Compound B (2.8 g; see Example 2) in methyl alcohol (4.5 ml) at 60°C. The reaction mixture was kept at 60°C for 5 hours and then cooled to 25°C and kept at 25°C for 10 hours.

37% hydrochloric acid (8 ml) was added to the reaction mixture, containing (4S,5R)-2-methyl-4-fluoromethyl-5-(4-methyl-sulfonylphenyl)-2-oxazoline (Compound D); the solution was refluxed for 4 hours and then cooled to 40°C. After removal of the solvents under vacuum at 40°C, the residue was taken up with methylene chloride (50 ml) and water (10 ml). After separation of the phases, the aqueous phase was extracted again with methylene chloride (2 x 50 ml) and the combined organic phases were dried over sodium sulfate and evaporated to dryness.

A residue was obtained containing (2S,3R)-3-(4-methylsulfonylphenyl)-3-hydroxy-2-amino-1-fluoro-propane (Compound E), which was dissolved in methyl alcohol (3 ml) and triethylamine (0.97 g; 96 mmoles); to the solution, kept at 25°C in an inert atmosphere, methyl dichloroacetate was added (1.6 g; 11.2 mmoles). The reaction mixture was heated to 40°C for 6 hours, the solvent was then evaporated under vacuum and the residue was taken up with methylene chloride (50 ml) and water (20 ml). After separation of the phases, the organic phase was dried over sodium sulfate and evaporated to dryness. A residue was thus obtained (2.6 g) which was crystallized from a solution of ethyl alcohol (2.2 ml) and water (0.4 ml) to give pure Florfenicol (2.02 g) whose chemical-physical properties were in accordance with the literature (EP 0 014 437).

### Example 5

### Florfenicol

A mixture of Compound C (1 g; 4.7 mmoles) in methyl alcohol (0.9 ml) and methyl chloroacetate (1.1 g; 7.7 mmoles) was refluxed for 1.5 hours. The mixture was cooled to 60°C and sodium tungstate (4 mg), EDTA (2 mg) and water (0.12 ml) were added.

130 volumes of hydrogen peroxide were dropped into the reaction mixture at 60°C. After completion of the dropwise addition, the reaction mixture was kept at 55°C for 2 hours. Afterwards, the reaction mixture was cooled to 0°C, the precipitate was filtered and washed with water.

700 mg of pure Fluorfenicol were obtained.

### Example 6

### (4S,5R)-2-phenyl-4-fluoromethyl-5-(4-methyl-thiophenyl)-2-oxazoline (Compound L)

To a solution of Compound C (1 g) in methylene chloride (5 ml) and triethylamine (0.47 g; 4.7 mmoles), kept at 25°C with stirring in an inert atmosphere, a solution of benzoyl chloride (0.56 g; 4.0 mmoles) in methylene chloride (2 ml) was added over 15 minutes. After 30 minutes (TLC analysis, eluent ethyl acetate : methyl alcohol = 8 : 2) an 8% solution of sodium bicarbonate (40 ml) was added to the reaction mixture. After separation of the phases, the organic phase was washed with 0.1N hydrochloric acid (5 ml) and water (5 ml). The organic phase was dried over sodium sulfate and the solvent was evaporated under vacuum.

A residue was obtained consisting of the N-benzoyl-derivatives of Compounds C and F in the ratio 1:1 (Compounds G and H, respectively), which was dissolved in methylene chloride (5 ml) and triethylamine (0.68 g; 6.7 mmoles). To the solution stirred at 25°C in an inert atmosphere, a solution of methanesulfonyl chloride (0.49; 3.50 mmoles) in methylene chloride (3 ml) was added.

After 1 hour, a solution of 8% sodium carbonate (40 ml) was added to the reaction mixture. After separation of the phases, the organic phase was washed with 0.1N hydrochloric acid (5 ml) and water (5 ml). The organic phase was dried over sodium sulfate and the solvent was evaporated under vacuum.

A residue was obtained which was chromatographed over silica gel to give pure Compound L (0.73 g) whose properties were in accordance with the literature (EP-A-0 130 633).

### Example 7

### Fluorthiomicamine Compound C)

A mixture of Compound L (15 g; 49.7 mmoles), water (17 ml) and 36% hydrochloric acid (28.5) was heated to 95°C for three hours. The mixture was cooled and the insoluble material was filtered under vacuum. The filtration waters were made alkaline to pH 8 with sodium bicarbonate and extracted with methylene chloride (50 ml x 2); the combined organic phases were dried over sodium sulfate and evaporated to dryness under vacuum 9.2 g of a crude product were obtained containing 70% of compound C.

Pure Compound C was obtained by crystallization from ethyl acetate.

### Example 8

### (2R,3S)-3-(4-methylthiophenyl)-3-hydrox-2-acetamidopropyl acetate (Compound N)

To solution of (2R,3S)-2-acetamido-3-(4-methylthiophenyl)-1,3-propanediol (Compound M) (5 g; 19.6 mmoles) in methylene chloride (50 ml), stirred at 25°C in an inert atmosphere, triethylamine (2.38 g; 23.5 mmoles) and acetyl chloride (1.7 g; 21.5 mmoles) were added. After 1 hour (TLC analysis; eluent, ethyl acetate) an 8% solution of sodium bicarbonate (40 ml) was added to the reaction mixture. After separation of the phases, the organic phase was washed with 0.5N hydrochloric acid (50 ml) and water (50 ml). The organic phase was dried over sodium sulfate and the solvent was evaporated under vacuum.

An oily residue was obtained which was crystallized from diethyl ether to give pure Compound N (4.2 g; 14.2 mmoles; yield, 73%).
¹H-NMR (300 MHz, CDCl₃) delta (ppm): 1.99 (s, 3H); 2.00 (s, 3H); 2.49 (s, 3H); 3.37 (d, J=3.8 Hz, 1H); 4.02 (dd, J=4.4 Hz, J=11.7 Hz, 1H); 4,25 (dd, J=7.5 Hz, J=11.7 Hz, 1H); 4.45 (dddd, J=4.4 Hz, J=7.5 Hz, J=4.1 Hz, J=8.5 Hz, 1H); 4.88 (dd, J=2.7 Hz, J=4.1 Hz, 1H); 5.98 (d, J=8.5 Hz, 1H); 7.20-7.30 (4H, aromatics).

### Example 9

### (4R,5R)-2-methyl-4-acetoxymethyl-5-(4-methyl-thiophenyl)-2-oxazoline (Compound O) and (4R,5R)-2-methyl-4-hydroxymethyl-5-(4-methylthiophenyl)-2-oxazoline (Compound P)

To a solution of Compound N (see Example 8; 3 g; 10.17 mmoles) in methylene chloride (15 ml) and triethylamine (2.54 g; 25.12 mmoles) kept at 25°C under nitrogen, a solution of mesyl chloride (1.5 g; 13.22 mmoles) in methylene chloride (10 ml) was added over 15 minutes. After 1 hour the reaction mixture was poured into an 8% sodium bicarbonate solution (10 ml). After separation of the phases, the organic phase was washed with 0.5N hydrochloric acid (15 ml) and water (5 ml). After evaporation of the solvent under vacuum, the residue was crystallized from diisopropyl ether (10 ml) to give pure Compound 0 (2.3 g; 8.24 mmoles; yield, 81%).
[alpha]D²⁰= -153.5° (c = 1, CDCl₃); m.p. = 64-66°C
¹H-NMR (CDCl₃) delta (ppm): 2.09 (s, 3H); 2.11 (d, J=1.11 Hz, 3H); 2.49 (s, 3H); 4.18 (m, 1H); 4.25 (m, 3H); 5.13 (d, J=6.82 Hz, 3H); 7.19-7.28 (4H, aromatics).
¹³C-NMR (CDCl₃): 14.0, 15.5, 15.6, 65.5, 73.4, 83.2, 126.1, 127.7, 136.7, 139.1, 165.8, 170.7.
IR (CCl₄): 1750, 1675, 1230 cm⁻¹.
MS: m/e (relative intensity): 280 (M+1; 41), 220 (47), 173 (11), 65 (100).

To a suspension of Compound O (2.3 g; 8.24 mmoles) in methyl alcohol (20 ml), sodium carbonate (1.38 g; 13 mmoles) was added at room temperature and with stirring. The reaction mixture was kept under stirring in an inert atmosphere at room temperature for 3 hours; methylene chloride (15 ml) was added to the suspension. After 0.5 hours, the solid was filtered and the filtration waters were evaporated to dryness under vacuum.

A solid was obtained which was crystallized from methyl alcohol to give 1.66 g (7.0 mmoles) of compound P.
¹H-NMR (CDCl₃) delta (ppm): 2.09 (d, J=1.41 Hz, 3H); 2.48 (s, 3H); 2.63 (dd, J=4.1 Hz, J=11.7 Hz, 1H); 3.91 (dd, J=8.0 Hz, J=11.7 Hz, 1H); 4.01 (dddq, J=4.1 Hz, J=8.0 Hz, J=7.8 Hz, J=1.4 Hz, 1H); 5.29 (d, J=7.8 Hz, 1H); 7.19-7.27 (4H, aromatics).

### Example 10

### (2R,3S)-3-(4-methylthiophenyl)-2-benzamido-1,3-propanediol (Compound Q) and (4R,5R)-2-phenyl-4-methane-sulfonyloxy-5-(4-methylthiophenyl)-2-oxazoline (Compound R)

To a solution of erythro (2R,3S)-2-amino-3-(4-methylthiophenyl)-1,3-propanediol (1.5 g; 7 mmoles) in dimethylformamide (4 ml) and triethylamine (0.76 g; 7.6 mmoles) stirred at 25°C in an inert atmosphere, a solution of benzoyl chloride (0.99 g; 7.05 mmoles) in dimethyl formamide (2 ml) was added over 15 minutes. After 1 hour (TLC analysis; eluent, ethyl acetate : methyl alcohol = 1 : 1), methylene chloride (30 ml) and an 8% solution of sodium bicarbonate (30 ml) were added to the reaction mixture. After separation of the phases the aqueous phase was extracted with methylene chloride (20 ml) and the combined organic phases were washed with 0,1N hydrochloric acid (10 ml) and water (10 ml). The organic phase was dried over sodium sulfate and the solvent was evaporated under vacuum.

A residue was obtained which was crystallized from isopropyl ether to give pure Compound Q (1.9 g; 6.1 mmoles; yield, 87%).
¹H-NMR (300 MHz, CDCl₃+D₂O) delta (ppm): 2.49 (s, 3H); 3.70 (dd, J=3.64 Hz, J=11.62 Hz, 1H); 3.92 (dd, J=3.17 Hz, J=11.62 Hz, 1H); 4.26 (dddd, J=3.17 Hz, J=3.64 Hz, J=3.59 Hz, J=8.10 Hz, 1H); 5.16 (d, J=3.59 Hz, 1H); 7.07 (d, J=8.10 Hz, 1H); 7.25-7.39 (4H, aromatics); 7.41-7.81 (5H, aromatics).

To a solution of Compound Q (1.9 g; 6.0 mmoles) in dimethyl formamide (6 ml), methylene chloride (3 ml) and triethylamine (670 mg; 6.6 mmoles), at room temperature under stirring and in an inert atmosphere, a solution of methanesulfonyl chloride (0.75 g; 6.6 mmoles) in methylene chloride (2 ml) was added.

After 1 hour (TLC analysis; eluent, diethyl ether) methylene chloride (10 ml) and sodium bicarbonate (20 ml) were added to the reaction mixture. After separation of the phases, the organic phase was washed with 0.1N hydrochloric acid (15 ml) and water (15 ml). The organic phase was dried over sodium sulfate and evaporated under vacuum to give Compound R whose chemical-physical properties were in accordance with the literature (EP-A-0 130 633).

### Example 11

### Compound A

Working in a manner similar to that described in EP-A-0 423 705.6 but starting from (2R,3S)-2-amino-3-(4-methylthiophenyl)-1,3-propanediol, (4R,5S)-2,2-dimethyl-3-acetyl-4-hydroxymethyl-5-(4-methylthiophenyl)-1,3-oxazoline (Compound B) was prepared.

To a solution of Compound S (15 g; 50.8 mmoles) and triethylamine (6.2 g; 61 mmoles) in methylene chloride (80 ml), methanesulfonyl chloride (6.35 g; 56 mmoles) was added under stirring at 15°C.

After 40 minutes the suspension was filtered under nitrogen and to the solution containing the 4-methanesulfonyloxy derivative of Compound S (Compound T), acetic anhydride (0.5 g) and a solution of methanesulfonic acid (10 g; 104 mmoles) in acetic anhydride (0.5 g; 5 mmoles) and methylene chloride (30 ml) were added at 20°C.

The reaction mixture was heated to 35°C for 1 hour and then poured into a solution of diethyl ether (200 ml) and triethylamine (17 g; 170 mmoles).

To the thus obtained solution water was added (150 ml). After separation of the phases, the aqueous phase was extracted with diethyl ether (50 ml). The combined organic phases were distilled under vacuum to give a residue (12.7 g) (Compound A). Compound T, which can be isolated after usual processing of the mixture with water and methylene chloride, has the following characteristics:
¹H-NMR (300 MHz, CDCl₃) delta (ppm): 1.71 (s, 3H); 1.75 (s, 3H); 2.21 (s, 3H); 2.49 (s, 3H); 2.64 (s, 3H); 3.57 (dd, J=6.93 Hz, J=10.53 Hz, 1H); 3.94 (dd, J=5.86 Hz, J=10.53 Hz, 1H); 4.34 (ddd, J=5.86 Hz, J=6.93 Hz, J=4.85 Hz, 1H); 5.33 (d, J=4.85 Hz, 1H); 7.25-7.31 (4H, aromatics).

### Example 12

### (4R,5R)-2-methyl-4-methanesulfonyloxymethyl-5-(4-methyl sulfonylphenyl)-2-oxazoline (Compound U)

To a solution of Compound A (1.0 g; 3.17 mmoles) in methyl alcohol (7 ml), heated to 40°C, sodium tungstate dihydrate (6.4 mg), ethylenediaminetetracetic acid disodium salt dihydrate (3 mg) and 35% hydrogen peroxide (0.85 ml, about 8.75 mmoles) were added.

The reaction mixture was kept at 40°C for 3 hours.

The reaction mixture was then concentrated to about 1/3 of the volume and methylene chloride (20 ml) and water (10 ml) were added.

The phases were separated and the organic phase was washed with water (10 ml). The organic phase was dried over sodium sulfate and evaporated to dryness under vacuum. A residue (0.6 g) mainly consisting of Compound U was obtained.
¹H-NMR (300 MHz, CDCl₃) delta (ppm): 2.16 (s, 3H); 3.06 (s, 3H); 3.09 (s, 3H); 4.2 (m, 1H); 4.35 (dd, J=10.5 Hz, J=5.95 Hz, 1H); 4.44 (dd, J=10.5 Hz, J=4.1 Hz, 1H); 5.44 (d, J=6.72 Hz, 1H); 7.50 (part BB' of the system AA'BB', J=8.3 Hz, 2H); 7.97 (part AA' of the system AA'BB', J=8.3 Hz, 2H).

Working in a similar manner but starting from oxazolines B, O and P, the analogous 4-methylsulfonylphenyl substituted compounds were obtained.

### Example 13

### (2R,3R)-2-amino-3-(4-methylthiophenyl)-1,3-propanediol (Thiomicamine)

Acetylation of Compound S (see Example 11) with acetyl chloride and triethylamine, affords (4R,5S)-2,2-dimethyl-3-acetyl-4-acetoxymethyl-5-(4-methylthiophenyl)-1,3-oxazolidine (Compound V).

To a solution of methanesulfonic acid (15 g; 0.156 mmoles), chloroform (45 ml) (ethanol free) and acetic anhydride (6.0 g; 0.058 mmoles), Compound V (20 g; 0.06 moles) was added under stirring at 25°C in a nitrogen atmospher.

The solution was heated to 35°C and kept at 35°C for 2 hours (solution A). Solution A was then poured at 15°C into a stirred solution of sodium hydroxide (19 g; 0.48 moles) in water (530 ml).

The mixture was heated under reflux while the mixture of water and chloroform (450 ml) was distilled. The solution was kept at 95°C for 4 hours, then cooled to 15°C for 2 hours. A precipitate was formed which was filtered, washed with water (2 x 10 ml) and dried under vacuum at 60°C to give pure Thiomicamine (10.7 g; 0.050 moles; yield, 85%).
[alpha]D²⁰=-33.2° (c = 2; 0.1N HCl); ee > 99%

In a parallel assay, solution A was poured into a solution of triethylamine (40.4 g; 0.40 moles) and ethyl ether (300 ml); water (100 ml) was added to the thus obtained suspension and, after separation of the phases, the organic phase was washed with water (100 ml), dried over sodium sulfate and evaporated to dryness.

A residue (17 g) was obtained which was 95.30% Compound O (see Example 9) while the remaining 4.7% was the (4R,5S) stereoisomer (Compound O').

### Example 14

### Compound O

To a solution of methanesulfonic acid (15 g; about 0.156 moles) and chloroform (45 ml) (ethanol free), Compound V (see Example 13) (20 g; 0.06 moles) was added under stirring at 25°C under nitrogen.

The solution was kept for 26 hours at 35°C and then poured into a stirred solution of diethyl ether (300 ml) and triethylamine (20.2 g; 0.2 moles). To the reaction mixture water (200 ml) was added; after separation of the phases, the organic phase was washed with 10% ammonium chloride (100 ml) and water (100 ml). The organic phases were dried over sodium sulfate and evaporated under vacuum. The residue (17 g) which contained compound 0 and the (4R,5S) stereoisomer thereof (Compound O') in the ratio 89:11 was crystallized from diisopropyl ether (200 ml) to give pure Compound O (13.2 g; 0.047 moles; yield, 79%).

The filtration waters were concentrated under vacuum and the residue was purified by flash chromatography (eluent, diethyl ether : triethyl amine = 97 : 3) to give pure Compound O' (0.84 g; 3 mmoles; yield, 5%), having the following characteristics:
[alpha]D²⁰= -132.1° (c = 1, CHCl₃); m.p. = 71-73°C
¹H-NMR (CDCl₃) delta (ppm): 1.92 (s, 3H); 2.13 (d, J=1.4 Hz, 3H); 2.48 (s, 3H); 3.62 (dd, J=6.14 Hz, J=16.7 Hz, 1H); 3.81 (dd, J=5.6 Hz, J=16.7 Hz, 1H); 4.52 (m, 1H); 5.60 (d, J=10.2 Hz, 1H); 7.10-7.30 (4H, aromatics).
¹³C-NMR (CDCl₃): 14.0, 15.6, 20.6, 64.0, 67.9, 82.1, 126.3, 126.5, 132.3, 138.9, 166.1, 170.4.
IR (CDCl₄): 1745, 1670, 1235 cm⁻¹.
MS: m/e (relative intensity): 280 (M+1; 7.22), 220 (18), 173 (5.5), 65 (100).

Compound O' (purity: greater than 99%) was treated at 35°C for 24 hours with methanesulfonic acid in chloroform containing acetic anhydride. After processing, a mixture of Compounds O and O' in the ratio 88.6 : 11.4 (NMR) was obtained.

### Example 15

### (4R,5R)-2-methyl-4-hydroxymethyl-5-(4-methyl-sulfonylphenyl)-2-oxazoline (Compound Z)

To a solution of Compound P (see Example 9; 1.5 g; 6.44 mmoles) in methyl alcohol (10.5 ml) stirred at 25°C, sodium tungstate dihydrate (12.5 mg), ethylene diamino tetracetic acid disodium salt dihydrate (5.85 mg) and 35% hydrogen peroxide (1.2 ml; 13.8 mmoles) were added.

The reaction mixture was kept at 25°C for 3 hours.

The reaction mixture was then concentrated to about 1/3 of its volume and methylene chloride (20 ml) and water (10 ml) were added.

The phases were separated and the organic phase was washed with water (10 ml). The organic phase was dried over sodium sulfate and evaporated to dryness under vacuum. A residue (1.5 g) was obtained mainly consisting of Compound Z.
¹H-NMR (300 MHz, CDCl₃-D₂O) delta (ppm): 2.14 (s, 3H); 3.05 (s, 3H); 3.73 (dd, J=4 Hz, J=11.7 Hz, 1H); 3.90 (dd, J=4.15 Hz, J=11.7 Hz, 1H); 4.00 (m, 1H); 7.49 (part B of the system AA'BB', J=7.8 Hz, 2H); 7.95 (part A of the system AA'BB', J=7.8 Hz, 2H).

## Claims

1. A process for preparing a 2-oxazoline compound of the formula 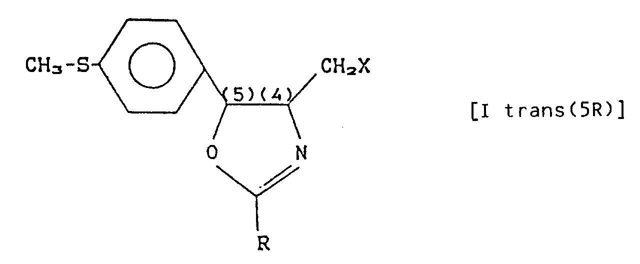 wherein
R is alkyl, alkoxy, alkyl substituted by 1 or 2 substituents selected from the group comprising halogens and C₁-C₄ alkoxy, alkenyl, alkenyl substituted by 1 or 2 substituents selected from the group comprising halogens and C₁-C₄ alkoxy, phenyl, phenyl substituted by 1 or 2 substituents selected from the group comprising halogens, C₁-C₄ alkoxy, C₁-C₄ alkyl, cyano, nitro and trifluoromethyl, phenylalkyl or phenylalkyl substituted by 1 or 2 substituents selected from the group comprising halogens, C₁-C₄ alkoxy, C₁-C₄ alkyl, cyano, nitro and trifluoromethyl, and
X is hydroxy, halogen or acyloxy from a carboxylic or sulfonic acid,
comprising treating a compound of the formula 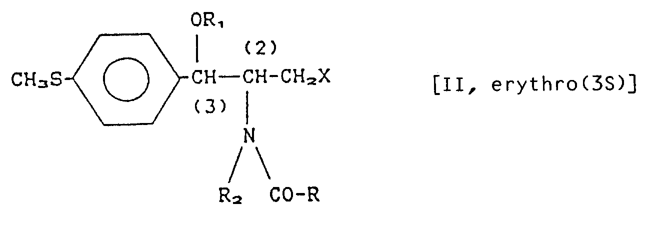 wherein
R and X have the above mentioned meanings,
R₁ is hydrogen or acyl,
R₂ is hydrogen, or
R₁ and R₂ together form a group 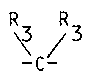 where each R₃ is hydrogen, alkyl, alkoxy or phenyl, or both R₃ together form a tetra or pentamethylene chain,
with an ionizing non-nucleophilic agent selected from strong protic acids and Lewis acids having affinity for oxygen, in a substantially anhydrous inert solvent or diluting agent, at a temperature of from -20 to 100°C.

2. A process according to claim 1, wherein both R₁ and R₂ are hydrogen in the compound of formula II.

3. A process according to claim 1, wherein in the compound of formula II R₁ and R₂ together form a group 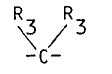 where each R₃ is methyl, ethyl, phenyl or together form a tetramethylene or pentamethylene chain.

4. A process according to any of the above claims wherein R is alkyl, alkyl substituted by 1 or 2 substituents selected from the group comprising halogens and C₁-C₄ alkoxy, phenyl or phenyl substituted by 1 or 2 substituents selected from the group comprising halogens, C₁-C₄ alkoxy, C₁-C₄ alkyl, cyano, nitro and trifluoromethyl

5. A process according to any of the above claims wherein X is fluoro, hydroxy, acetoxy, benzoyloxy, propanoyloxy, methanesulfonyloxy, ethanesulfonyloxy, benzenesulfonyloxy or p-toluenesulfonyloxy.

6. A process according to claim 1 wherein the ionizing non-nucleophilic agent is selected from the group comprising trifluoroacetic acid, sulfuric acid, methanesulfonic acid, p-toluenesulfonic acid, trifluoromethanesulfonic acid, AlCl₃, and protonated tertiary amines.

7. A process according to claim 1, wherein the ionizing non-nucleophilic agent comprises methanesulfonyl chloride and a tertiary amine, when R₁ is OH in the formula II.

8. A process according to claim 1, wherein the inert solvent and the inert diluent are selected from the group comprising chlorinated hydrocarbons, aromatic hydrocarbons and ethers.

9. A process according to claim 1, wherein the temperature is of from 0 to 40°C.

## Patentansprüche

1. Verfahren zur Herstellung einer 2-Oxazolin-Verbindung der Formel 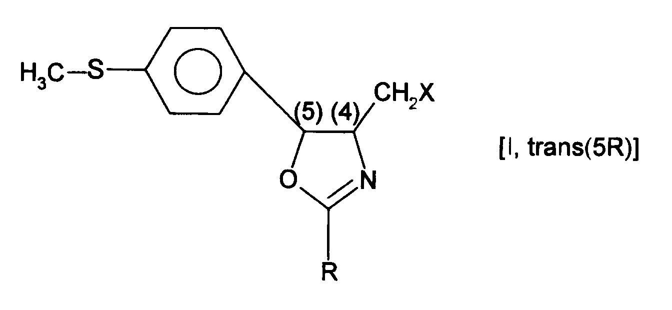 worin
R für Alkyl, Alkoxy, Alkyl, das mit 1 oder 2 Substituenten, ausgewählt unter Halogenen und C₁-C₄-Alkoxy, substituiert ist, Alkenyl, Alkenyl, das mit 1 oder 2 Substituenten, ausgewählt unter Halogenen und C₁-C₄-Alkoxy, substituiert ist, Phenyl, Phenyl, das mit 1 oder 2 Substituenten, ausgewählt unter Halogenen, C₁-C₄-Alkoxy, C₁-C₄-Alkyl, Cyano, Nitro und Trifluormethyl, substituiert ist, Phenylalkyl oder Phenylalkyl, das mit 1 oder 2 Substituenten, ausgewählt unter Halogenen, C₁-C₄-Alkoxy, C₁-C₄-Alkyl, Cyano, Nitro und Trifluormethyl, substituiert ist, steht und
X für Hydroxy, Halogen oder Acyloxy einer Carbon- oder Sulfonsäure steht,
umfassend die Umsetzung einer Verbindung der Formel 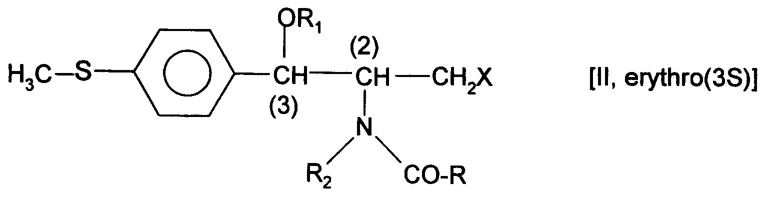 worin
R und X die oben genannten Bedeutungen besitzen,
R₁ für Wasserstoff oder Acyl steht,
R₂ für Wasserstoff steht, oder
R₁ und R₂ gemeinsam eine Gruppe 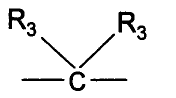 bilden, wobei jede Gruppe R₃ für Wasserstoff, Alkyl, Alkoxy oder Phenyl steht, oder beide Gruppen R₃ gemeinsam eine Tetra- oder Pentamethylenkette bilden,
mit einem ionisierenden nichtnucleophilen Agens, ausgewählt unter starken protischen Säuren und Lewissäuren mit einer Affinität für Sauerstoff, in einem im Wesentlichen wasserfreien inerten Lösungsmittel oder Verdünnungsmittel, bei einer Temperatur von -20 bis 100 °C.

2. Verfahren nach Anspruch 1, worin R₁ und R₂ in der Verbindung der Formel II für Wasserstoff stehen.

3. Verfahren nach Anspruch 1, worin R₁ und R₂ in der Verbindung der Formel II gemeinsam eine Gruppe 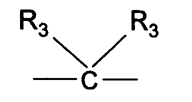 bilden, worin jede Gruppe R₃ für Methyl, Ethyl, Phenyl steht oder beide gemeinsam für eine Tetramethylen- oder Pentamethylenkette stehen.

4. Verfahren nach einem der vorhergehenden Ansprüche, worin R für Alkyl, Alkyl, das mit 1 oder 2 Substituenten, ausgewählt unter Halogenen und C₁-C₄-Alkoxy, substituiert ist, Phenyl oder Phenyl, das mit 1 oder 2 Substituenten, ausgewählt unter Halogenen, C₁-C₄-Alkoxy, C₁-C₄-Alkyl, Cyano, Nitro und Trifluormethyl, substituiert ist, steht.

5. Verfahren nach einem der vorhergehenden Ansprüche, worin X für Fluor, Hydroxy, Acetoxy, Benzoyloxy, Propanoyloxy, Methansulfonyloxy, Ethansulfonyloxy, Benzolsulfonyloxy oder p-Toluolsulfonyloxy steht.

6. Verfahren nach Anspruch 1, wobei das ionisierende nichtnucleophile Agens ausgewählt ist unter Trifluoressigsäure, Schwefelsäure, Methansulfonsäure, p-Toluolsulfonsäure, Trifluormethansulfonsäure, AlCl₃ und protonierten tertiären Aminen.

7. Verfahren nach Anspruch 1, worin das ionisierende nichtnucleophile Agens Methansulfonylchlorid und ein tertiäres Amin umfasst, wenn R₁ in Formel II für OH steht.

8. Verfahren nach Anspruch 1, wobei das inerte Lösungsmittel und das inerte Verdünnungsmittel ausgewählt sind unter Chlorkohlenwasserstoffen, aromatischen Kohlenwasserstoffen und Ethern.

9. Verfahren nach Anspruch 1, wobei die Temperatur 0 bis 40 °C beträgt.

## Revendications

1. Procédé de préparation d'un composé de la 2-oxazoline de formule 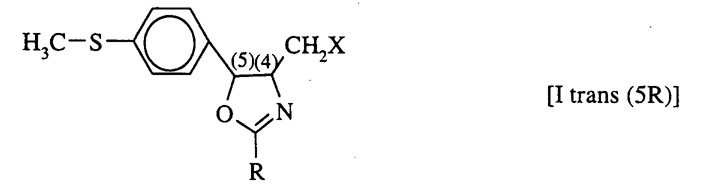
où R est un alkyl, un alcoxy, un alkyle substitué par un ou deux substituants choisis dans le groupe comprenant les halogènes et les alcoxy en C₁-C₄, un alcényle, un alcényle substitué par un ou deux substituants choisis dans le groupe comprenant les halogènes et les alcoxy en C₁-C₄, un phényle, un phényle substitué par un ou deux substituants choisis dans le groupe comprenant les halogènes, les alcoxy en C₁-C₄, les alkyle en C₁-C₄, le cyano, le nitro et le trifluorométhyle, un phénylalkyle ou un phénylalkyle substitué par un ou deux substituants choisis dans le groupe comprenant les halogènes, les alcoxy en C₁-C₄, les alkyle en C₁-C₄, le cyano, le nitro et le trifluorométhyle et X est un hydroxyle, un halogène ou un reste acyloxy d'un acide carboxylique ou sulfonique,
comprenant le traitement d'un composé de formule 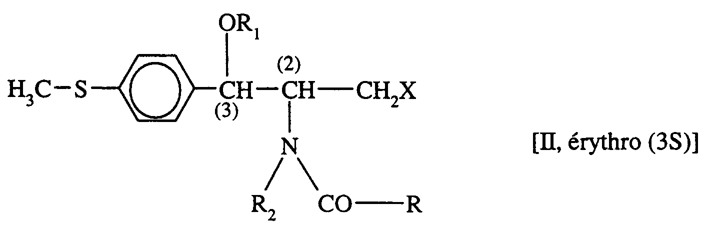
où R et X ont les significations indiquées ci-dessus,
R₁ est un hydrogène ou un acyle,
R₂ est un hydrogène ou
R₁ et R₂, considérés dans leur ensemble, forment un groupe 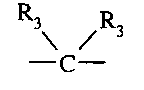 où chaque R₃ est un hydrogène, un alkyle, un alcoxy ou un phényle ou où les deux R₃, considérés dans leur ensemble, forment une chaîne tétra- ou pentaméthylène,
par un agent ionisant non nucléophile choisi parmi les acides protiques et les acides de Lewis forts ayant une affinité pour l'oxygène, dans un solvant ou un agent diluant inerte sensiblement anhydre, à une température de -20 à 100°C.

2. Procédé selon la revendication 1, dans lequel R₁ et R₂ sont tous deux un hydrogène dans le composé de formule II.

3. Procédé selon la revendication 1, dans lequel dans le composé de formule II, R₁ et R₂ forment ensemble un groupe 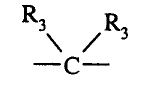 où chaque R₃ est un méthyle, un éthyle ou un phényle ou forment ensemble une chaîne tétraméthylène ou pentaméthylène.

4. Procédé selon une quelconque des revendications précédentes où R est un alkyle, un alkyle substitué par un ou deux substituants choisis dans le groupe comprenant les halogènes et les alcoxy en C₁-C₄, un phényle ou un phényle substitué par un ou deux substituants choisis dans le groupe comprenant les halogènes, les alcoxy en C₁-C₄, les alkyle en C₁-C₄, le cyano, le nitro et le trifluorométhyle.

5. Procédé selon une quelconque des revendications précédentes où X est fluoro, hydroxy, acétoxy, benzoyloxy, propanoyloxy, méthanesulfonyloxy, éthanesulfonyloxy, benzènesulfonyloxy ou p-toluènesulfonyloxy.

6. Procédé selon la revendication 1, dans lequel l'agent ionisant non nucléophile est choisi dans le groupe comprenant l'acide trifluoroacétique, l'acide sulfurique, l'acide méthanesulfonique, l'acide p-toluènesulfonique, l'acide trifluorométhanesulfonique, AlCl₃ et les amines tertiaires protonées.

7. Procédé selon la revendication l, dans lequel l'agent ionisant non nucléophile comprend le chlorure de méthanesulfonyle et une amine tertiaire, quand R₁ est OH dans la formule II.

8. Procédé selon la revendication 1, dans lequel le solvant inerte et le diluant inerte sont choisis dans le groupe comprenant les hydrocarbures chlorés, les hydrocarbures aromatiques et les éthers.

9. Procédé selon la revendication 1, dans lequel la température est de 0 à 40°C.
